# FASCICULE DE BREVET EUROPEEN

(11) **EP 1 019 101 B1**
(45) Date de publication et mention de la délivrance du brevet: **27.03.2002**
(21) Numéro de dépôt: 97901741.5
(22) Date de dépôt: 13.02.1997
(51) Int. Cl.: A61L 11/00

(54) **PROCEDE ET ENSEMBLE DE STERILISATION DE DECHETS CONTAMINES**
VERFAHREN UND VORRICHTUNG ZUR STERILISATION VON INFEKTIONSMÜLL
METHOD AND ASSEMBLY FOR STERILISING CONTAMINATED WASTE

(30) Priorité: 15.02.1996 FR 9602096
(43) Date de publication de la demande: 19.07.2000
(73) Titulaire: Medical Dual System, 92100 Boulogne Billancourt (FR)
(72) Inventeur: Levy, Alfred, 92100 Boulogne (FR); Bettoun, Guy, 92100 Boulogne (FR)
(74) Mandataire: Jacquard, Philippe Jean-Luc
(86) Numéro de dépôt international: IB9700123
(87) Numéro de publication internationale: WO9729791

(56) Documents cités:
- WO-A-90/14847
- WO-A-90/15419
- WO-A-93/12841
- US-A- 4 552 720
- US-A- 4 662 516

## Description

La présente invention concerne un ensemble de stérilisation et de transformation de déchets contaminés, et notamment des déchets médicaux contaminés, tels que par exemple, des aiguilles, scalpels, et seringues usagés.

Dans le domaine du traitement des déchets contaminés et notamment des déchets médicaux contaminés, il est connu, par exemple d'utiliser des incinérateurs puissants ou des dispositifs de stérilisation permettant de faire fondre les parties métalliques des déchets contaminés, par exemple, les aiguilles et les scalpels, avec un arc électrique ou une décharge électrique. Ces derniers dispositifs sont relativement lourds et nécessitent une source d'alimentation électrique, telle qu'une batterie ou une prise de courant, ce qui limite leur portabilité et leur disponibilité à des hôpitaux. De tels dispositifs sont décrits par exemple dans les documents EP-A-0 634 182, WO 94/13346, et WO 94/01153.

Toutefois, ces dispositifs ne peuvent pas généralement être installés ou utilisés par des professionnels de la santé, tels que médecins et infirmières, en dehors d'un milieu hospitalier. La législation en vigueur concernant le stockage et le traitement de déchets contaminés leur pose ainsi un problème constant et non négligeable. En effet, le problème de portabilité et de fonctionnement de tout dispositif de stérilisation doit être pris en compte pour ces personnels de santé, en profession libérale par exemple, ou en médecine ambulatoire.

Par ailleurs, la demande WO-A-90/15419 décrit un dispositif de traitement par stérilisation de déchets médicaux ou contaminés, comportant un récipient, dont une partie est compressible, à l'intérieur duquel est disposé au moins un composé thermofusible. Dans un premier temps, le récipient est chauffé dans un four, éventuellement muni d'un filtre pour les gaz libérés lors du traitement thermique, pour faire fondre le composé thermofusible autour des déchets, les rendant ainsi méconnaissables et stériles. Dans un deuxième temps, le récipient est comprimé pour permettre l'englobage des éléments coupants formant partie des déchets à stériliser, qui risqueraient de transpercer le récipient, s'ils n'étaient pas complètement englobés.

L'inconvénient de cette solution est que le récipient formant enceinte n'est pas intègre, c'est-à-dire n'est pas suffisamment résistant d'un point de vue mécanique, car déformable par compression, ce qui représente un danger potentiel pour les tiers, si le récipient venait à s'ouvrir, par exemple lors de son ramassage par le camion à ordures. Par ailleurs, l'utilisation d'un filtre sur la paroi du four pose également de nombreux inconvénients, à savoir :
- la nécessité de disposer d'un four fermé de manière hermétique afin de justifier l'existence ;
- l'entretien nécessaire du filtre pour éviter son colmatage ;
- le risque d'une infection accidentelle lors d'un tel entretien par des microorganismes ayant pu se loger dans celui-ci ;
- le risque, en cas de colmatage, d'une explosion de l'ensemble du fait d'une pression trop importante de gaz libérés dans l'enceinte du four ;
- ainsi que la pollution inévitable de l'environnement lors de l'ouverture du four, car contenant des vapeurs potentiellement dangereuses.

L'objet de la présente invention concerne donc un ensemble de stérilisation et de transformation, permettant à un particulier, tel qu'un médecin ou infirmière en cabinet médical, à la fois de stocker provisoirement les déchets contaminés, y compris métalliques, et de les rendre stériles et non réutilisables en toute sécurité, avec un équipement simple et facile à mettre en oeuvre. Les déchets ainsi transformés par l'ensemble selon la présente invention peuvent être jetés avec les ordures ménagères ou industrielles sans risque de contamination pour une tierce personne qui pourrait entrer en contact avec les déchets ainsi traités.

Conformément à l'invention, on prévoit un ensemble jetable de collecte de déchets contaminés, comprenant un récipient thermostable, par exemple en métal, pour supporter sans se déformer les conditions, notamment température, d'une stérilisation complète de son contenu, et destiné aux ordures ménagères et/ou industrielles, formant une enceinte munie d'une ouverture obturable par laquelle sont introduits les déchets contaminés, et une matière plastique thermofusible dans les conditions, notamment température, de la stérilisation, disposée à l'intérieur du récipient, avec une masse suffisante pour englober les déchets dans la matière plastique fondue, l'ensemble étant plus particulièrement caractérisé en ce qu'il comporte en outre un bouchon perméable, comportant un filtre, obturant l'ouverture du récipient.

L'ensemble selon la présente invention est destiné à une utilisation en cabinet médical. Il résulte de cette utilisation que l'ensemble selon l'invention se doit d'être complet, et notamment en garantissant l'intégrité du récipient dans les conditions de stérilisation, pour fonctionner en toute sécurité, sans émanation olfactive. Il n'était donc pas acceptable d'utiliser un ensemble de traitement comprenant un récipient qui laisserait échapper des émanations olfactives dans l'atmosphère, ou qui présenterait des risques d'infection accidentelle, ou d'explosion comme dans l'ensemble de l'art antérieur précédemment discuté. Selon l'ensemble de la présente invention, le récipient, y compris le bouchon muni d'un filtre, est à usage unique, ce qui veut dire que les problèmes de l'art antérieur ne se présentent pas. En effet, le récipient, fermé par son bouchon comportant le filtre, est simplement jeté après stérilisation des déchets. Ceci a un autre avantage en ce que l'on effectue, également une stérilisation du filtre, sans être obligé d'y toucher, ce qui accroît la sécurité de l'ensemble.

Conformément à un autre objet de l'invention, on prévoit un procédé de stérilisation de déchets contaminés comprenant les étapes suivantes consistant à :
- introduire des déchets contaminés dans un récipient jetable, formant enceinte de stérilisation, constitué d'une matière thermostable dans les conditions, notamment température de stérilisation, et pendant la durée prédéterminée opérationnelle de la stérilisation, le récipient contenant une matière plastique thermofusible dans lesdites conditions de stérilisation ;
- obturer le récipient irréversiblement, avant de le placer dans le four, par encliquetage avec un bouchon comportant un filtre ;
- placer le récipient contenant les déchets contaminés dans un four adapté spécifiquement pour fournir lesdites conditions de stérilisation, pour assurer la stérilisation complète des déchets contaminés ;
- chauffer le four dans lesdites conditions de stérilisation, dé façon à ce que la matière plastique thermofusible disposée dans le récipient jetable fonde et englobe les déchets, rendant ainsi le récipient jetable et les déchets non réutilisables ;
- retirer du four le récipient, contenant les déchets stérilisés et englobés par la matière plastique thermofusible, et jeter ledit récipient.

De préférence, le bouchon est agencé pour être rapporté sur le récipient, de manière à l'obturer irréversiblement.

Avantageusement, la température prédéterminée de stérilisation est située entre 50°C et 300°C, de préférence entre 200°C et 300°C, et la durée prédéterminée de stérilisation est comprise entre 15 et 120 minutes, de préférence entre 15 et 90 minutes.

Dans un mode d'exécution préférée de l'invention, le bouchon est pourvu d'une partie mâle, constituée par un jonc, pouvant s'accoupler de manière irréversible avec une partie femelle, constituée par une collerette déformable élastiquement, ménagée sur le récipient.

Dans un mode d'exécution préféré, la matière plastique thermofusible est disposée sur les parois intérieures du récipient jetable.

Dans un autre mode d'exécution préféré de l'invention, la matière plastique thermofusible est disposée en bloc au fond du récipient jetable.

Avantageusement, la matière plastique thermofusible est une résine ou cire synthétique ou naturelle thermofusible à une température comprise entre 50°C et 300°C, de préférence entre 200° et 300°C, et choisie parmi le groupe consistant en les cires, les résines oléfiniques, les résines phénoliques, les résines vinyliques, et les résines acryliques et/ou un mélange de celles-ci.

Dans un mode d'exécution préféré de l'invention, le récipient jetable comporte également un témoin révélateur de stérilisation disposé à l'extérieur du récipient. Le témoin révélateur de stérilisation peut être constitué par une fine lamelle contenant un réactif coloré thermosensible, dont la modification est visible à l'oeil nu, disposée sur une paroi extérieure du récipient.

Avantageusement, l'ouverture est en outre obturée de manière réversible par un couvercle, solidaire de, et mobile en rotation par rapport au récipient, le couvercle comportant lui-même une ouverture pouvant venir en registre avec l'ouverture du récipient, afin d'assurer l'ouverture et l'obturation du récipient. De préférence, le couvercle comporte également des encoches de préhension d'objets tranchants et/ou piquants.

Dans un mode d'exécution préféré de l'invention, l'ensemble comprend également un four comportant des moyens de réglage de la température et de la durée prédéterminées de stérilisation, et fixant ladite température à une plage comprise entre 50°C et 300°C, de préférence entre 200°C et 300°C, et ladite durée à une plage comprise entre 15 et 120 minutes, de préférence entre 15 et 90 minutes.

Plus préférentiellement, le four comporte des moyens de verrouillage de la porte du four pendant ladite durée prédéterminée, et de déverrouillage automatique dès que la température interne du four est suffisamment basse pour permettre la manipulation du récipient sans danger pour l'utilisateur.

La présente invention sera plus particulièrement illustrée par la description détaillée suivante d'un exemple non limitatif, accompagnée par les dessins en annexe, dans lesquels :
- la Figure 1 représente une vue éclatée, partiellement en coupe transversale, d'un ensemble selon l'invention, avant stérilisation, le bouchon n'étant pas utilisé et mis de côté ;
- la Figure 2 représente le même ensemble en coupe transversale, prêt à être stérilisé, avec son ouverture obturée de manière irréversible par un bouchon formant filtre ;
- la Figure 3 représente le même ensemble en coupe transversale, après stérilisation.

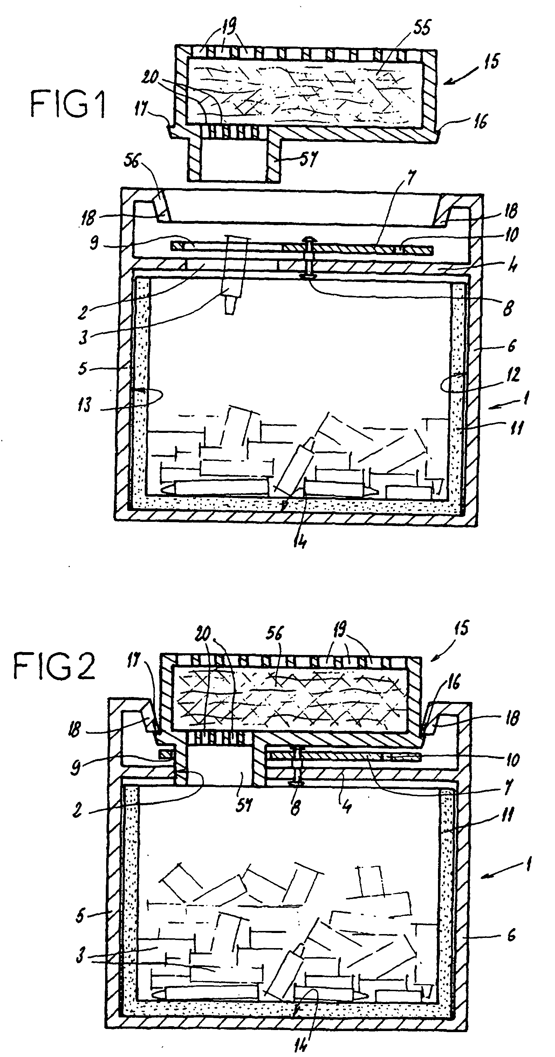

Conformément à la Figure 1, l'ensemble comprend un récipient 1, jetable formant enceinte de stérilisation, muni d'une ouverture 2 obturable par laquelle sont introduits les déchets 3 contaminés. Le récipient 1 jetable est constitué d'une matière thermostable et étanche, par exemple en un métal tel que l'acier inoxydable, permettant de maintenir l'intégrité du système de stérilisation à toutes les étapes de son utilisation. En effet, le récipient doit être thermostable et indéformable, aux conditions de stérilisation, notamment aux températures relativement importantes pendant toute la durée nécessaire et suffisante pour la stérilisation, mais également à l'état refroidi. Pour cette raison, le récipient sera de préférence constitué d'acier inoxydable, d'une épaisseur d'environ 0,26 mm, possédant l'homologation "Autorisation de Transport de Matières Dangereuses" selon la norme européenne du "Groupe I Solide". Le récipient constitue donc en lui-même une barrière absolue vis-à-vis des objets coupants ou piquants contenus dans son intérieur. La forme du récipient n'est pas très importante, mais sera de préférence de forme cylindrique ou parallélépipédique. L'ouverture 2 est pratiquée dans la paroi supérieure 4 du récipient, ou peut être une pièce rapportée, fixée par sertissage ou soudage aux parois latérales 5, 6 du récipient. De préférence l'ouverture 2 est circulaire et de diamètre compris entre 1 cm et 7 cm, de préférence 5 cm. L'ouverture 2 sert également de cheminée d'évacuation pour les gaz dégagés pendant la stérilisation, comme décrit ci-après.

Cette ouverture 2 est fermée par un couvercle 7 du type "boîte à sel", qui est solidaire du récipient 1, et mobile en rotation par rapport à ce dernier grâce à un axe 8 qui traverse le couvercle 7 et la paroi supérieure 4 du récipient 1. Le couvercle 7 à la forme d'une galette, disposée parallèlement à la paroi supérieure 4, et comporte sa propre ouverture 9, pouvant venir en registre avec celle 2 du récipient 1, par rotation du couvercle 7, ce qui permet l'accès au récipient pour l'introduction des déchets contaminés, et la fermeture de l'ouverture 2 de celui-ci. Le couvercle 7 est également muni d'au moins une encoche 10, permettant sa préhension directement avec des objets coupants et/ou piquants tels que des aiguilles et des scalpels, sans manipulation et donc sans risque de blessure accidentelle.

Dans une variante d'exécution (non représentée), les déchets peuvent être introduits dans le récipient par une ouverture de 1 à 10 cm de diamètre ménagée directement dans la paroi supérieure. Cette ouverture est agencée de telle sorte que l'on puisse introduire les déchets dans le récipient, sans toutefois pouvoir les ressortir, par exemple en prévoyant une ouverture à clapet anti-retour. Une deuxième ouverture ou encoche peut également être ménagée dans la paroi supérieure ou le clapet afin de permettre la préhension d'objets coupants et/ou piquants tels des aiguilles ou scalpels.

Le récipient 1 contient, disposé à l'intérieur, un garnissage 11, par exemple ayant la forme d'un seau, en matière plastique thermofusible à la température de la stérilisation. De préférence, le garnissage est dispose à proximité ou sur les parois intérieures 12, 13, ou encore uniquement en bloc sur le fond 14 du récipient jetable 1. La matière plastique thermofusible est par exemple une résine synthétique ou naturelle, thermofusible à une température comprise entre 50 et 300°C, et de préférence entre 200° et 300°C, et est choisie parmi le groupe consistant en les résines oléfiniques, les résines phénoliques, les résines vinyliques, et les résines acryliques et/ou un mélange de celles-ci. Une des fonctions de cette matière plastique est, en fondant, d'englober les déchets, et de transférer par conduction la température de stérilisation aux déchets, de telle sorte que ceux-ci, même englobés, se stérilisent et se transforment dans la masse fondue de la matière plastique, les rendant ainsi, d'une part non identifiables et inoffensifs, et d'autre part non réutilisables. Les déchets deviennent ainsi méconnaissables, ce qui évite la reconnaissance d'un déchet psychologiquement agressif à la perception visuelle par une tierce personne, et existent sous forme d'un palet solidaire et adhérant aux parois du récipient 1, ce qui supprime le risque d'éparpillement de son contenu en cas de rupture ou ouverture accidentelle.

Par ailleurs, pour augmenter la sécurité d'utilisation de l'ensemble en cabinet médical, la résine présente une autre fonction qui est celle d'absorber presque complètement les vapeurs dégagées par la stérilisation. Ainsi, et de manière préférée, on a retenu comme résine une cire microcristalline, présentant :
- un point de fusion compris entre 69 et 76°C (selon norme française NFT-60.121) ;
- et une viscosité à 10°C de 7,5 à 8 cSt (selon norme française NFT-60.100).

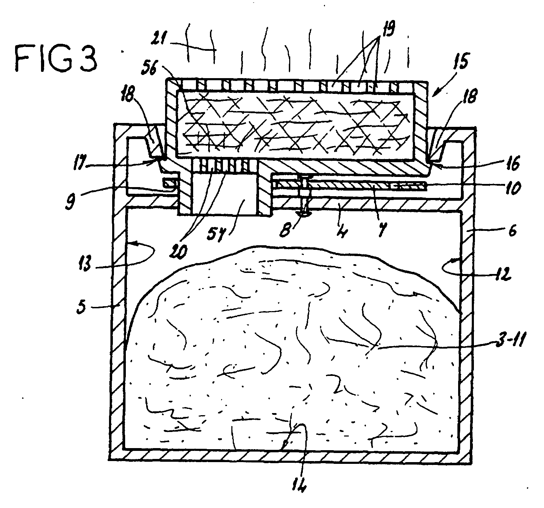

L'ensemble comporte en outre un bouchon 15 perméable comportant un filtre 55. Ce bouchon 15 peut être de toute forme appropriée, mais sera de préférence de forme cylindrique ou parallélépipédique et complémentaire et adaptée pour une fixation définitive dans le col 18 du récipient 1, ménagé et disposé au-dessus de la paroi supérieure 4. Le filtre 55 sert à traiter des gaz dégagés par la stérilisation, de manière à piéger et retenir les effluents toxiques ou nuisibles vis-à-vis de l'environnement, résultant de la stérilisation. Il est à noter que le filtre est également stérilisé pendant le traitement, ce qui augmente la sécurité du système. De préférence, le filtre est en un matériau résistant aux conditions de stérilisation, par exemple en laine de roche, et non en charbon activé comme le filtre utilisé dans l'art antérieur, car le déposant a découvert que ce dernier type de filtre cesse d'exercer une activité filtrante aux températures de stérilisation. Le bouchon 15 comporte une partie mâle, comprenant un jonc 16, qui s'accouple de manière irréversible, par exemple par encliquetage, avec une partie femelle, constituée par une collerette 18 continue, déformable élastiquement, ménagée dans un col disposé et au-dessus de la paroi supérieure 4, et de l'ouverture 2. Le bouchon 15 est muni d'ouvertures 19, 20, ménagées à sa partie supérieure et à sa partie inférieure. Une fois le bouchon 15 assemblé dans le col du récipient 1 un joint étanche est créé, et il n'est plus possible de dissocier l'un de l'autre, ce qui empêche par ailleurs tout échappement de matière solide. Cette situation est représentée par les Figures 2 et 3, et plus particulièrement par la Figure 3, dans laquelle on voit que des gaz d'échappement 21 s'évacuent du récipient 1, à travers le filtre 55.

Le bouchon 15 comprend, par exemple, mais non obligatoirement, une cheminée inférieure 57, servant à son indexation par rapport à l'ouverture 2 ménagée dans la partie supérieure 4 du récipient 1. Cette cheminée 57 est agencée pour pénétrer dans l'ouverture 2, au travers de l'ouverture 9 du couvercle 7, dans la position assemblée et définitive du bouchon 15 dans le col 18. Les passages 20 du bouchon 15 établissent la communication requise entre la cheminée 57 et l'intérieur dudit bouchon, comprenant le filtre 55.

Dans un autre mode d'exécution, le bouchon 15 comprenant le filtre 55 peut venir coiffer le récipient 1 et se visser de manière irréversible, ou bien se souder au récipient par le biais d'un matière thermodurcissable disposé sur la collerette 18 ou le jonc 16.

Le récipient 1 peut également être muni d'un témoin révélateur de stérilisation, connu en soi et non représenté, disposé à l'extérieur du récipient. De préférence, le témoin révélateur de stérilisation est constitué par une fine lamelle contenant un réactif coloré thermosensible, dont la modification est visible à l'oeil nu, disposée sur une paroi extérieure du récipient, par exemple, par collage. De tels témoins sont disponibles dans le commerce, et vendus notamment par la Société Ansell Medical. Ces témoins garantissent le niveau de stérilisation à l'intérieur du récipient, et sont couramment utilisés dans les hôpitaux pour la stérilisation des outils et matériels chirurgicaux.

L'ensemble de stérilisation est associé à un four, qui n'est pas représenté dans les figures, car sa forme est celle d'un four traditionnel, à usage domestique, et ne nécessite pas de remarque particulière. Il suffit simplement que le four soit de dimensions plus importantes que l'ensemble précédemment décrit, avec le bouchon 15. Le four est spécifiquement adapté pour fournir une température opérationnelle prédéterminée, pendant une durée prédéterminée, suffisante pour assurer la stérilisation complète des déchets contaminés. De préférence, le four comporte des moyens de réglage de la température et de la durée prédéterminées de stérilisation, qui fixent ladite température dans une plage comprise entre 50°C et 300°C, de préférence entre 200°C et 300°C, et ladite durée dans une plage comprise entre 15 et 120 minutes, et de préférence entre 15 et 60 minutes. Le four peut également comporter des moyens de verrouillage de la porte du four pendant ladite durée prédéterminée, et de déverrouillage automatique dès que la température interne du four est suffisamment basse pour permettre la manipulation du récipient sans danger pour l'utilisateur.

Le procédé conforme à l'invention sera maintenant décrit, par référence à un utilisateur, par exemple un médecin travaillant en cabinet, ou devant se déplacer.

Au départ, le bouchon 15 est maintenu à l'écart, de telle sorte que le récipient 1 est directement utilisable, en ouvrant ou fermant l'ouverture 2 grâce au couvercle rotatif 7.

Les déchets contaminés sont introduits au fur et à mesure de l'exercice du médecin dans le récipient à travers les ouvertures 2 et 9. Au terme du délai de stockage autorisé par la loi ou si le récipient 1 est rempli avant ce terme, le médecin ferme le récipient 1 de manière irréversible par encliquetage du bouchon 15 sur le col 18. A cette fin, l'ouverture 2 est maintenue ouverte par l'alignement en registre, de cette dernière et de l'ouverture 9 du couvercle, traversées par la cheminée 57. L'ensemble ainsi fermé est placé dans le four. Ce dernier est ensuite chauffé à environ 230°C pendant 15 à 120, de préférence entre 15 et 60 minutes, pour stériliser le contenu du récipient 1, et faire fondre le garnissage 11 thermofusible, rendant ainsi le récipient et les déchets stérilisés et non réutilisables. Au terme de la durée prédéterminée, le médecin vérifie que la stérilisation est achevée par le changement de couleur du témoin révélateur, ou, si le four est équipé de moyens le permettant, par ouverture automatique de la porte du four. Le récipient ainsi stérilisé, et son contenu, ayant pris la forme d'un palet solide amalgamant les déchets solides et la matière plastique thermofusible, peuvent alors être jetés directement dans les ordures ménagères ou industrielles, sans nécessité d'effectuer un traitement ultérieur de stérilisation, et sans risque vis-à-vis de l'environnement.

## Revendications

1. Ensemble jetable de collecte de déchets contaminés, comprenant :
- un récipient (1) thermostable, par exemple en métal, pour supporter sans se déformer les conditions, notamment température, d'une stérilisation complète de son contenu, et destiné aux ordures ménagères et/ou industrielles, formant une enceinte munie d'une ouverture (2) obturable par laquelle sont introduits les déchets (3) contaminés ;
- et une matière plastique (11) thermofusible dans les conditions, notamment température, de la stérilisation, disposée à l'intérieur du récipient (1), avec une masse suffisante pour englober les déchets (3) dans la matière plastique fondue ;
**caractérisé en ce que** l'ensemble comporte en outre un bouchon perméable (15), comportant un filtre (55), obturant l'ouverture (2) du récipient (1).

2. Ensemble selon la revendication 1, **caractérisé en ce que** le bouchon (15) est agencé pour être rapporté sur le récipient (1), de manière à l'obturer irréversiblement.

3. Ensemble selon la revendication 1 ou la revendication 2, **caractérisé en ce que** le bouchon (15) est pourvu d'une partie mâle, constituée par un jonc (16), pouvant s'accoupler de manière irréversible avec une partie femelle, constituée par une collerette (56) déformable élastiquement, ménagée sur le récipient (1).

4. Ensemble selon la revendication 1, **caractérisé en ce que** la matière plastique (11) thermofusible est disposée sur les parois intérieures (5,6) du récipient (1) jetable.

5. Ensemble selon la revendication 1, **caractérisé en ce que** la matière plastique thermofusible est disposée en bloc au fond (14) du récipient (1) jetable.

6. Ensemble selon la revendication 1, **caractérisé en ce que** la matière plastique (11) thermofusible est une résine ou cire synthétique ou naturelle thermofusible à une température comprise entre 50°C et 300°C, de préférence entre 200° et 300°C, et choisie parmi le groupe consistant en les cires, les résines oléfiniques, les résines phénoliques, les résines vinyliques, et les résines acryliques et/ou un mélange de celles-ci.

7. Ensemble selon la revendication 1, **caractérisé en ce que** la matière plastique (11) thermofusible est une cire microcristalline, présentant un point de fusion compris entre 69 et 76°C (selon la norme française NFT-60.121), et une viscosité à 10°C de 7,5 à 8 cSt (selon la norme française NFT-60.100).

8. Ensemble selon l'une quelconque des revendications précédentes, **caractérisé en ce que** le récipient jetable comporte également un témoin révélateur de stérilisation disposé à l'extérieur du récipient.

9. Ensemble selon la revendication 8, **caractérisé en ce que** le témoin révélateur de stérilisation est constitué par une fine lamelle contenant un réactif coloré thermosensible, dont la modification est visible à l'oeil nu, disposée sur une paroi extérieure du récipient.

10. Ensemble selon l'une quelconque des revendications 1 à 8, **caractérisé en ce que** l'ouverture (2) est en outre obturée, de manière réversible, par un couvercle (7), solidaire de, et mobile en rotation par rapport au récipient (1), le couvercle (7) comportant lui-même une ouverture (9) pouvant venir en registre avec l'ouverture (2) du récipient (1), afin d'assurer l'ouverture et l'obturation du récipient (1).

11. Ensemble selon la revendication 10, **caractérisé en ce que** le couvercle (7) comporte également des encoches (10) de préhension d'objets tranchants et/ou piquants.

12. Ensemble selon l'une quelconque des revendications précédentes, **caractérisé en ce qu'**il comporte en outre un four comportant des moyens de réglage de la température et de la durée prédéterminées de stérilisation, et fixant ladite température à une plage comprise entre 50°C et 300°C, de préférence entre 200°C et 300°C, et ladite durée à une plage comprise entre 15 et 120 minutes, de préférence entre 15 et 90 minutes.

13. Ensemble selon la revendication 12, **caractérisé en ce que** le four comporte des moyens de verrouillage de la porte du four pendant ladite durée prédéterminée, et de déverrouillage automatique dès que la température interne du four est suffisamment basse pour permettre la manipulation du récipient sans danger pour l'utilisateur.

14. Procédé de stérilisation de déchets contaminés comprenant les étapes suivantes consistant à :
- introduire des déchets (3) contaminés dans un récipient (1) jetable, formant enceinte de stérilisation, constitué d'une matière thermostable dans les conditions, notamment température de stérilisation, et pendant la durée prédéterminée opérationnelle de la stérilisation, le récipient (1) contenant une matière plastique (11) thermofusible dans lesdites conditions de stérilisation ;
- obturer le récipient (1) irréversiblement, avant de le placer dans le four, par encliquetage avec un bouchon (15) comportant un filtre (55) ;
- placer le récipient (1) contenant les déchets contaminés (3) dans un four adapté spécifiquement pour fournir lesdites conditions de stérilisation, pour assurer la stérilisation complète des déchets contaminés (3) ;
- chauffer le four dans lesdites conditions de stérilisation, de façon à ce que la matière plastique (11) thermofusible disposée dans le récipient (1) jetable fonde et englobe les déchets (3), rendant ainsi le récipient (1) jetable et les déchets (3) non réutilisables ;
- retirer du four le récipient (1), contenant les déchets (3) stérilisés et englobés par la matière plastique (11) thermofusible, et jeter ledit récipient.

15. Procédé selon la revendication 14, **caractérisé en ce que** la température prédéterminée de stérilisation est située entre 50°C et 300°C, de préférence entre 200°C et 300°C.

16. Procédé selon la revendication 14, **caractérisé en ce que** la durée prédéterminée de stérilisation est comprise entre 15 et 120 minutes, de préférence entre 15 et 90 minutes.

## Patentansprüche

1. Wegwerfbare Gesamtheit von einer Sammlung von kontaminierten Abfällen, umfassend:
- einen wärmestabilen Behälter (1), z.B. aus Metall, zum Aushalten, ohne sich zu deformieren, der Bedingungen, insbesondere der Temperatur, einer vollständigen Sterilisation seines Inhalts, und bestimmt für Haushaltsmüll und/oder industriellen Müll, welcher eine Umgrenzung bildet, die mit einer verschließbaren Öffnung (2) versehen ist, durch welche die kontaminierten Abfälle (3) eingeführt werden;
- und ein Plastik- bzw. Kunststoffmaterial (11), das unter den Bedingungen, insbesondere der Temperatur, der Sterilisation wärmeschmelzbar ist, welches in dem Inneren des Behälters (1) mit einer Masse angeordnet ist, die zum Einschließen der Abfälle (3) in dem geschmolzenen Plastik- bzw. Kunststoffmaterial genügend ist;
**dadurch gekennzeichnet, dass** die Gesamtheit außerdem einen permeablen Verschluss (15) hat, der ein Filter (55) enthält, welcher die Öffnung (2) des Behälters (1) verschließt.

2. Gesamtheit gemäß Anspruch 1, **dadurch gekennzeichnet, dass** der Verschluss (15) dazu eingerichtet ist, um in der Art und Weise auf den Behälter (1) angefügt zu werden, dass er ihn irreversibel verschließt.

3. Gesamtheit gemäß Anspruch 1 oder Anspruch 2, **dadurch gekennzeichnet, dass** der Verschluss (15) mit einem erhabenen bzw. vorstehenden Teil versehen ist, der durch eine Wulst bzw. Sicke (16) gebildet ist, welcher sich mit einem vertieften bzw. zurückspringenden Teil in irreversibler Art und Weise kuppeln bzw. verbinden kann, der von einem elastisch deformierbaren Bund bzw. Kragen (56) gebildet ist, welcher auf dem Behälter (1) angebracht bzw. ausgebildet ist.

4. Gesamtheit gemäß Anspruch 1, **dadurch gekennzeichnet, dass** das wärmeschmelzbare Plastik- bzw. Kunststoffmaterial (11) auf den Innenwänden (5, 6) des wegwerfbaren Behälters (1) angeordnet ist.

5. Gesamtheit gemäß Anspruch 1, **dadurch gekennzeichnet, dass** das wärmeschmelzbare Plastik- bzw. Kunststoffmaterial im Block bzw. als Ganzes auf dem Boden (14) des wegwerfbaren Behälters (1) angeordnet ist.

6. Gesamtheit gemäß Anspruch 1, **dadurch gekennzeichnet, dass** das wärmeschmelzbare Plastik- bzw. Kunststoffmaterial (11) ein synthetisches oder natürliches Harz oder Wachs ist, das bei einer Temperatur zwischen 50°C und 300°C, vorzugsweise zwischen 200° und 300°C, wärmeschmelzbar und aus der Gruppe ausgewählt ist, die aus den Wachsen, den Olefinharzen, den Phenolharzen, den Vinylharzen und den Acrylharzen und/oder einer Mischung derselben ausgewählt ist.

7. Gesamtheit gemäß Anspruch 1, **dadurch gekennzeichnet, dass** das wärmeschmelzbare Plastik- bzw. Kunststoffmaterial (11) ein mikrokristallines Wachs ist, das einen Schmelzpunkt zwischen 69 und 76°C (gemäß der französischen Norm NFT-60.121) aufweist, sowie eine Viskosität bei 10°C von 7,5 bis 8 cSt (gemäß der französischen Norm NFT-60.100).

8. Gesamtheit gemäß irgendeinem der vorhergehenden Ansprüche, **dadurch gekennzeichnet, dass** der wegwerfbare Behälter außerdem einen Kontrollentwickler bzw. - anzeiger für die Sterilisation enthält, der im bzw. am Äußeren des Behälters angeordnet ist.

9. Gesamtheit gemäß Anspruch 8, **dadurch gekennzeichnet, dass** der Kontrollentwickler bzw. -anzeiger für die Sterilisation von einer feinen Lamelle bzw. einem feinen Plättchen gebildet ist, die bzw. das ein gefärbtes wärmeempfindliches Reagenz enthält, dessen Modifikation mit dem bloßen Auge sichtbar ist, welche bzw. welches auf einer Außenwand des Behälters angeordnet ist.

10. Gesamtheit gemäß irgendeinem der Ansprüche 1 bis 8, **dadurch gekennzeichnet, dass** die Öffnung (2) außerdem in reversibler Art und Weise durch eine Abdeckung (7) verschlossen ist, die verbunden bzw. fest verbunden mit dem und drehbeweglich mit Bezug auf den Behälter (1) ist, wobei die Abdeckung (7) selbst eine Öffnung (9) hat, die in Übereinstimmung mit der Öffnung (2) des Behälters (1) kommen kann, um die Öffnung und den Verschluss des Behälters (1) sicherzustellen.

11. Gesamtheit gemäß Anspruch 10, **dadurch gekennzeichnet, dass** die Abdeckung (7) außerdem Aus- bzw. Einschnitte (10) für das Erfassen von schneidenden und/oder stechenden Objekten hat.

12. Gesamtheit gemäß irgendeinem der vorhergehenden Ansprüche, **dadurch gekennzeichnet, dass** sie außerdem einen Ofen hat, der Mittel für die Regulierung bzw. Regelung der Temperatur und der vorbestimmten Dauer der Sterilisation hat und die genannte Temperatur in einem Bereich, der zwischen 50°C und 300°C, vorzugsweise zwischen 200°C und 300°C ist, sowie die genannte Dauer in einem Bereich, der zwischen 15 und 120 Minuten, vorzugsweise zwischen 15 und 90 Minuten, ist, fixiert.

13. Gesamtheit gemäß Anspruch 12, **dadurch gekennzeichnet, dass** der Ofen Mittel für die Verriegelung der Tür des Ofens während der genannten vorbestimmten Dauer und für die automatische Entriegelung, sobald die Innentemperatur des Ofens genügend niedrig ist, um die Manipulation des Behälters ohne Gefahr für den Benutzer zu gestatten, hat.

14. Verfahren für die Sterilisation von kontaminierten Abfällen, umfassend die folgenden Schritte, bestehend aus:
- Einführen der kontaminierten Abfälle (3) in einen wegwerfbaren Behälter (1), der eine Umgrenzung für die Sterilisation bildet, welcher von einem Material gebildet ist, das unter den Bedingungen, insbesondere der Temperatur der Sterilisation, und während der vorbestimmten operativen Dauer der Sterilisation wärmestabil ist, wobei der Behälter (1) ein Plastik- bzw. Kunststoffmaterial (11) enthält, das unter den genannten Bedingungen der Sterilisation wärmeschmelzbar ist;
- irreversibles Verschließen des Behälters (1) vor dessen Platzierung in dem Ofen durch Verklinkung bzw. Sperrung mit einem Verschluss (15), der ein Filter (55) enthält;
- Platzieren des Behälters (1), welcher die kontaminierten Abfälle (3) enthält, in einem Ofen, der spezifisch zum Liefern der genannten Sterilisationsbedingungen angepasst bzw. geeignet ist, um die vollständige Sterilisation der kontaminierten Abfälle (3) sicherzustellen;
- Heizen des Ofens unter den genannten Sterilisationsbedingungen in der Art und Weise, dass das wärmeschmelzbare Plastik-bzw. Kunststoffmaterial (11), welches in dem wegwerfbaren Behälter (1) angeordnet ist, schmilzt und die Abfälle (3) einschließt, was so den wegwerfbaren Behälter (1) und die Abfälle (3) nicht wiederverwendbar macht;
- Herausnehmen des Behälters (1), der die sterilisierten und durch das wärmeschmelzbare Plastik- bzw. Kunststoffmaterial (11) eingeschlossenen Abfälle (3) enthält, aus dem Ofen und Wegwerfen des genannten Behälters.

15. Verfahren gemäß Anspruch 14, **dadurch gekennzeichnet, dass** die vorbestimmte Temperatur der Sterilisation zwischen 50°C und 300°C, vorzugsweise zwischen 200°C und 300°C, liegt.

16. Verfahren gemäß Anspruch 14, **dadurch gekennzeichnet, dass** die vorbestimmte Dauer der Sterilisation zwischen 15 und 120 Minuten, vorzugsweise zwischen 15 und 90 Minuten, liegt.

## Claims

1. Disposable assembly for collecting contaminated waste, comprising:
- a heat-stable container (1), for example made of metal, in order to withstand, without deformation, the conditions, particularly the temperature conditions, of a complete sterilization of its contents, and intended for household and/or industrial refuse, and forming a chamber provided with a sealable opening (2) through which the contaminated waste (3) is introduced;
- and a hot-melt plastic material (11) which is meltable under the conditions, particularly the temperature conditions, of sterilization, and is arranged inside the container (1), with a sufficient mass to enclose the waste (3) in the molten plastic material;
**characterized in that** the assembly additionally includes a permeable cover (15), with a filter (55), sealing the opening (2) of the container (1).

2. Assembly according to claim 1, **characterized in that** the cover (15) is designed to be fitted on the container (1) in such a way as co seal it irreversibly.

3. Assembly according to claim 1 or claim 2, **characterized in that** the cover (15) is provided with a male part, consisting of a retainer ring (16), which can engage irreversibly with a female part, consisting of an elastically deformable flange (56) formed on the container (1).

4. Assembly according to claim 1, **characterized in that** the hot-melt plastic material (11) is arranged on the inner walls (5, 6) of the disposable container (1).

5. Assembly according to claim 1, **characterized in that** the hot-melt plastic material is arranged en bloc on the bottom (14) of the disposable container (1).

6. Assembly according to claim 1, **characterized in that** the hot-melt plastic material (11) is a synthetic or natural resin or wax which is meltable at a temperature of between 50°C and 300°C, preferably between 200° and 300°C, and is chosen from the group consisting of waxes, olefinic resins, phenolic resins, vinyl resins and acrylic resins and/or a mixture of these.

7. Assembly according to claim 1, **characterized in that** the hot-melt plastic material (11) is a microcrystalline wax having a melting point of between 69 and 76°C (according to French standard NFT-60.121) and a viscosity at 10°C of 7.5 to 8 cSt (according to French standard NFT-60.100).

8. Assembly according to any one of the preceding claims, **characterized in that** the disposable container also includes a sterilization indicator arranged outside the container.

9. Assembly according to claim 8, **characterized in that** the sterilization indicator consists of a thin plate containing a heat-sensitive color reagent whose change is visible to the naked eye, said plate being arranged on an outer wall of the container.

10. Assembly according to any one of claims 1 to 8, **characterized in that** the opening (2) is additionally sealed in a reversible manner by a lid (7) which is integral with the container (1) and can be moved in rotation with respect thereto, the lid (7) itself including an opening (9) which can be brought into register with the opening (2) of the container (1) in order to ensure the opening and sealing of the container (1).

11. Assembly according to claim 10, **characterized in that** the lid (7) also includes notches (10) for gripping cutting and/or piercing articles.

12. Assembly according to any one of the preceding claims, **characterized in that** it additionally includes an oven including means for regulating the predetermined temperature and duration of sterilization, and fixing said temperature at a range of between 50°C and 300°C, preferably between 200°C and 300°C, and fixing said duration at a range of between 15 and 120 minutes, preferably between 15 and 90 minutes.

13. Assembly according to claim 12, **characterized in that** the oven includes means for locking the door of the oven during said predetermined duration, and for automatically unlocking the door once the temperature inside the oven is sufficiently low to allow the container to be handled without danger to the user.

14. Method for sterilizing contaminated waste, comprising the following steps, which consist in:
- introducing contaminated waste (3) into a disposable container (1) forming a sterilization chamber, made of a heat-stable material which is stable under the conditions, particularly the temperature conditions, of sterilization, and for the predetermined operating period of sterilization, the container (1) containing a hot-melt plastic material (11) which is meltable under said conditions of sterilization;
- sealing the container (1) irreversibly, before placing it in the oven, by snap-fitting a cover (15) which includes a filter (55);
- placing the container (1), containing the contaminated waste (3), in an oven specifically adapted to provide said conditions of sterilization, in order to ensure complete sterilization of the contaminated waste (3);
- heating the oven under said conditions of sterilization in such a way that the hot-melt plastic material (11) arranged in the disposable container (1) melts and encloses the waste (3), thereby making the disposable container (1) and the waste (3) non-reusable;
- removing from the oven the container (1) containing the waste (3) which has been sterilized and enclosed by the hot-melt plastic material (11), and disposing of said container.

15. Method according to claim 14, **characterized in that** the predetermined sterilization temperature is situated between 50°C and 300°C, preferably between 200°C and 300°C.

16. Method according to claim 14, **characterized in that** the predetermined duration of sterilization is between 15 and 120 minutes, preferably between 15 and 90 minutes.
